# EUROPEAN PATENT SPECIFICATION

(11) **EP 3 068 411 B1**
(45) Date of publication and mention of the grant of the patent: **18.03.2020**
(21) Application number: 14861625.3
(22) Date of filing: 12.11.2014
(51) Int. Cl.: A61K 35/76, A61K 35/765, A61K 35/768, A61P 35/00, A61K 31/337, A61K 31/555

(54) **ONCOLYTIC VIRUSES AND INCREASED CANCER TREATMENT REGIMENS**
ONKOLYTISCHE VIREN UND WIRKSAMERE KREBSBEHANDLUNGSSCHEMATA
VIRUS ONCOLYTIQUES ET SCHÉMAS POSOLOGIQUES DE TRAITEMENT DU CANCER RENFORCÉS

(30) Priority: 15.11.2013 US 201361904783 P
(43) Date of publication of application: 21.09.2016
(73) Proprietor: Oncolytics Biotech Inc., Calgary, AB T2N 1X7 (CA)
(72) Inventor: COFFEY, Matthew C., Calgary, Alberta T2N 3L4 (CA); THOMPSON, Bradley G., Calgary, Alberta T2N 0Z5 (CA)
(74) Representative: Haseltine Lake Kempner LLP
(86) International application number: PCT/CA2014/000806
(87) International publication number: WO 2015/070323

(56) References cited:
- WO-A1-2009/143610
- SEI SHIZUKO ET AL: "Synergistic antitumor activity of oncolytic reovirus and chemotherapeutic agents in non-small cell lung cancer cells", MOLECULAR CANCER, BIOMED CENTRAL, LONDON, GB, vol. 8, no. 1, 14 July 2009 (2009-07-14), page 47, XP021058735, ISSN: 1476-4598, DOI: 10.1186/1476-4598-8-47
- E. M. KARAPANAGIOTOU ET AL: "Phase I/II Trial of Carboplatin and Paclitaxel Chemotherapy in Combination with Intravenous Oncolytic Reovirus in Patients with Advanced Malignancies", CLINICAL CANCER RESEARCH, vol. 18, no. 7, 1 April 2012 (2012-04-01), pages 2080-2089, XP055348630, US ISSN: 1078-0432, DOI: 10.1158/1078-0432.CCR-11-2181
- MANISH R. PATEL ET AL: "Oncolytic virus therapy for cancer: the first wave of translational clinical trials", TRANSLATIONAL RESEARCH, vol. 161, no. 4, 1 April 2013 (2013-04-01) , pages 355-364, XP055077880, ISSN: 1931-5244, DOI: 10.1016/j.trsl.2012.12.010
- CLEMENTS D. ET AL.: 'Reovirus in cancer therapy: an evidence based review.' ONCOLYTIC VIROTHERAPY vol. 3, 2014, pages 69 - 82, XP055343576
- KANAI R. ET AL.: 'Oncolytic herpes simplex virus vectors and chemotherapy: are combinatorial strategies more effective for cancer?''.' FUTURE ONCOLOGY vol. 6, no. 4, April 2010, pages 619 - 634, XP055343578

## Description

### BACKGROUND OF THE INVENTION

Cancer is one of the leading causes of death. Although it has been the focus of medical research for a long period of time, the main cancer therapies to date are surgery, radiation therapy and chemotherapy. Each one of these therapies is subject to limitations including, for example, the differing effects of the same therapy to subjects with similar types of cancer.

### BRIEF SUMMARY OF THE DISCLOSURE

The invention is defined in and by the appended claims. The present disclosure provides herein a method of treating cancer in a subject. The method includes administering to the subject an oncolytic virus and an increased treatment regimen of a chemotherapeutic agent, wherein administration treats the cancer in the subject. Also provided is a kit that includes an oncolytic virus and a chemotherapeutic agent, wherein the chemotherapeutic agent comprises an amount for an increased treatment regimen of a chemotherapeutic agent. An increased treatment regime is increased in amount (dosage, frequency or duration) as compared to standard therapy.

The details of one or more embodiments are set forth in the accompanying description below. Other features, objects, and advantages will be apparent from the description and from the claims.

### DETAILED DESCRIPTION OF THE DISCLOSURE

The present disclosure provides herein a method of treating cancer in a subject. The method includes administering to the subject an oncolytic virus and an increased treatment regimen of a chemotherapeutic agent as compared to standard therapy, wherein administration treats the cancer in the subject. Notably, the treatment is effective despite the deviation from standard therapy. In the provided methods, administration of an increased treatment regimen of a chemotherapeutic agent is permissible at least in part due to the reduction or prevention of toxicity (e.g., neurotoxicity) usually associated with chemotherapy as a result of adminsitration of the oncolytic viruses.

Optionally, the increased treatment regimen comprises an increased number of doses per treatment cycle of the chemotherapeutic agent as compared to standard therapy or comprises an increased amount per dose of the chemotherapeutic agent as compared to standard therapy. Optionally, the increased treatment regimen comprises an increased amount per dose and an increased number of doses of the chemotherapeutic agent as compared to standard therapy. Optionally, the increased treatment regimen comprises an increased number of treatment cycles with the chemotherapeutic agent as compared to standard therapy. Optionally, the increased number of treatment cycles comprises more than four treatment cycles with the chemotherapeutic agent. Optionally, the increased number of treatment cycles comprises 5, 6, 7, 8, 9, 10, 11, 12, 13, 14, 15, 16, 17, 18, 19, 20, 21, 22, 23, 24, 25 or more treatment cycles with the chemotherapeutic agent. The appended claims specify that the oncolytic virus is for use in a method comprising administering to the subject an increased number of treatment cycles with a chemotherapeutic agent as compared to standard therapy, wherein the increased number of treatment cycles comprises 9, 10, 11, 12, 13, 14, 15, 16, 17, 18, 19, 20, 21, 22, 23, 24, 25 or more treatment cycles with the chemotherapeutic agent.

As used herein, the term "chemotherapy" refers to the treatment of cancer with one or more cytotoxic agents (e.g., chemotherapeutic agents) according to a treatment regimen. As used herein, a treatment regimen refers to a protocol under which chemotherapy is given to a subject for treatment of disease. The term "induction treatment regimen" refers to a chemotherapy regimen used for the initial treatment of a disease while a "maintenance treatment regimen" refers to the ongoing use of chemotherapy to reduce the chances of a cancer reoccurance or prevention of continued cancer growth. As used herein, chemotherapeutic agents include natural, semisynthetic, or synthetic substances that work through a cytotoxic mechanism to result in apoptosis or cell death. A chemotherapeutic treatment regimen can include administration to a subject of one chemotherapeutic agent or a combination of chemotherapeutic agents. Chemotherapeutic agents include, but are not limited to, alkylating agents, anthracyclines, taxanes, epothilones, histone deacetylase inhibitors, inhibitors of Topoisomerase I, inhibitors of Topoisomerase II, kinase inhibitors, monoclonal antibodies, nucleotide analogs and precursor analogs, peptide antibiotics, platinum-based compounds, retinoids, and vinca alkaloids and derivatives.

As used herein, "standard therapy" or "standard treatment regimen" refers to the standard of care used (e.g., by a practicing physician) for the treatment of a disease, e.g., cancer. By way of example, a standard treatment regimen or standard therapy for a particular agent, e.g., agent X, can be that the agent is given for each cycle of treatment at a dose of 10 mg/kg once a week and up to four cycles of treatment are given with the cycles of treatment being given on consecutive or non-consecutive weeks. Alternatively, a standard treatment regimen can be administration of agent X at a dose of 10 mg/kg once a week for four weeks and up to six cycles of treatment can be given. Thus, agent X can be given for four weeks at the dose of 10 mg/kg once a week up to six times and the cycles of treatment can be given consecutively or non-consecutively. The time between cycles of treatment can vary and can be from one or more hours, one or more days, one or more weeks, or one or more months. The particular treatment regimens or standard therapy for a particular drug, e.g., a chemotherapeutic agent will vary. For example, the standard treatment regimen or standard therapy can vary based on the stage of cancer being treated, the class of patient being treated, whether the patient has been previously treated with a cancer therapy, and the like. Standard therapy for a particular chemotherapeutic agent can be found in the prescribing information guidelines for that chemotherapeutic agent as approved by the Federal Drug Adminsitraiton (FDA) or other regulatory authority, e.g., European Medicines Agency (EMA), Health Canada, and the like. Standard therapies for a given cancer can also be found at http://www.clinicalpharmacology-ip.com/help/find_and_list/lists/combo_chemo_regimens_list.htm. Thus, for example, an increased treatment regimen of a chemotherapeutic agent is an increase over the amount or dose given, the number of times the agent is given per treatment cycle and/or the number treatment cycles given as set forth in the presecribing information for a particular cheomtherapeutic agent or combination of chemotherapeutic agents.

As used herein, the phrase "increased treatment regimen" refers to a treatment regimen or prescribed course of medical treatment that is increased as compared to the treatment regimen or prescribed course of medical treatment given under standard therapy or standard treatment regimens. Thus, an increased treatment regimen as compared to standard therapy or a standard treatment regimen refers to an increased dose, an increased number of times a dose or agent is prescribed or given or an increased number of treatment cycles an agent is prescribed or given. Optionally, standard therapy includes a reduction in the amount (e.g., dose) or frequency of an agent that is prescribed or given, e.g., due to the presence of or increase in one or more adverse side effect such as toxcitity (e.g., neurotoxicity). Thus, an increased treatment regimen includes a regimen that does not involve reduction in the amount or frequency of an agent that is prescribed or given, e.g., due to the absence of or lack of increase in one or more adverse side effects typically observed upon administration of the agent. By way of example, the standard therapy for a subject with non-small cell lung cancer using a combination of carboplatin and paclitaxel is paclitaxel administered as a 3 hour intravenous infusion at a dose of 200 mg/m2 and carboplatin then administered as a 30 minute intravenous infusion at a dose calculated by the Calvert formula (AUC 6 mg/mL minute with GFR measured by 51Cr EDTA) on day 1 of a 21 day cycle. This cycle is repeated every 21 days for a total of up to four cycles of treatment. However, the standard therapy for a subject with non-small cell lung cancer using a combination of carboplatin and paclitaxel that is responsive to the four cycle treatment is up to six cycles of treatment. Thus, in the provided methods, a combination of carboplatin and paclitaxel is administered as an increased treatment regimen if more than four or more than six cycles of treatment are given and/or if one or both of the carboplatin or paclitaxel is administered at a dose greater than AUC 6 mg/mL or greater than 200 mg/m2, respectively, and/or if one or both of the carboplatin or paclitaxel is administered more than one time in a treatment cycle.

As used herein, the term "cancer" refers to all types of cancer, proliferative disorders, neoplasia, or malignant tumors found in mammals, including lymphomas, leukemias, blastomas, germ cell tumors, carcinomas and sarcomas. Exemplary cancers include cancer of the brain, breast, cervix, colon, head and neck, liver, kidney, lung, non-small cell lung, melanoma, mesothelioma, ovary, sarcoma, stomach, uterus and medulloblastoma. Optionally, the cancer is a neoplasm. Optionally, the cancer is head and neck cancer. Optionally, the cancer is lung cancer, liver cancer, lymphoma, pancreatic cancer, melanoma, kidney cancer or ovarian cancer. Optionally, as discussed in more detail below, the cancer is ras-activated.

Optionally, the cancer is metastatic. As used herein, the terms "metastasis," "metastatic," and "metastatic cancer" can be used interchangeably and refer to the spread of a proliferative disease or disorder, e.g., cancer, from one organ or another non-adjacent organ or body part. Cancer occurs at an originating site, e.g., breast, which site is referred to as a primary tumor, e.g., primary breast cancer. Some cancer cells in the primary tumor or originating site acquire the ability to penetrate and infiltrate surrounding normal tissue in the local area and/or the ability to penetrate the walls of the lymphatic system or vascular system circulating through the system to other sites and tissues in the body. A second clinically detectable tumor formed from cancer cells of a primary tumor is referred to as a metastatic or secondary tumor. When cancer cells metastasize, the metastatic tumor and its cells are presumed to be similar to those of the original tumor. Thus, if lung cancer metastasizes to the breast, the secondary tumor at the site of the breast consists of abnormal lung cells and not abnormal breast cells. The secondary tumor in the breast is referred to a metastaticlung cancer. Thus, the phrase metastatic cancer refers to a disease in which a subject has or had a primary tumor and has one or more secondary tumors. The phrases non-metastatic cancer or subjects with cancer that is not metastatic refers to diseases in which subjects have a primary tumor but not one or more secondary tumors. For example, metastatic lung cancer refers to a disease in a subject with or with a history of a primary lung tumor and with one or more secondary tumors at a second location or multiple locations, e.g., in the breast.

Oncolytic viruses that are used in the provided methods and kits include, but are not limited to, oncolytic viruses that are members in the family of myoviridae, siphoviridae, podpviridae, teciviridae, corticoviridae, plasmaviridae, lipothrixviridae, fuselloviridae, poxyiridae, iridoviridae, phycodnaviridae, baculoviridae, herpesviridae, adnoviridae, papovaviridae, polydnaviridae, inoviridae, microviridae, geminiviridae, circoviridae, parvoviridae, hepadnaviridae, retroviridae, cyctoviridae, reoviridae, birnaviridae, paramyxoviridae, rhabdoviridae, filoviridae, orthomyxoviridae, bunyaviridae, arenaviridae, leviviridae, picornaviridae, sequiviridae, comoviridae, potyviridae, caliciviridae, astroviridae, nodaviridae, tetraviridae, tombusviridae, coronaviridae, glaviviridae, togaviridae, and barnaviridae. Immunoprotected viruses and reassortant or recombinant viruses of these and other oncolytic viruses are also encompassed by the provided methods. Thus, the oncolytic virus used in the provided methods is, for example, selected from the group consisting of a reovirus, a Newcastle disease virus (NDV), a vesicular stomatitis virus (VSV), an adenovirus, a vaccinia virus, a parapox orf virus, a Sindbis virus, and a herpes simplex virus. Furthermore, a combination of at least two oncolytic viruses can also be employed to practice the provided methods. A few oncolytic viruses are discussed below, and a person of ordinary skill in the art can practice the present methods using additional oncolytic viruses as well according to the disclosure herein and knowledge available in the art. The appended claims specify that the oncolytic virus is a reovirus having IDAC Accession No. 190907-01.

In cancers for which oncolytic virus is used as a treatment, one or more of the proliferating cells associated with the cancer may have a mutation in which the ras gene (or an element of the ras signaling pathway) is activated, either directly (e.g., by an activating mutation in ras) or indirectly (e.g., by activation of an upstream or downstream element in the ras pathway). Activation of an upstream element in the ras pathway includes, for example, transformation with epidermal growth factor receptor (EGFR) or Sos. See, for example, Wiessmuller and Wittinghofer, 1994, Cellular Signaling 6(3):247-267; and Barbacid, 1987, Ann. Rev. Biochem. 56, 779-827. Activation of a downstream element in the ras pathway includes, for example, mutation within B-Raf. See, for example, Brose et al., 2002, Cancer Res. 62:6997-7000. A cancer or proliferative disorder that results, at least in part, by the activation of ras, an upstream element of ras, or an element in the ras signaling pathway is referred to herein as a ras-mediated proliferative disorder or cancer. In addition, the oncolytic virus is useful for treating proliferative disorders caused by mutations or dysregulation of PKR. See, for example, Strong et al., 1998, EMBO J. 17:3351-62. Normally, when a virus enters a cell, double-stranded RNA Kinase (PKR) is activated, blocking protein synthesis, and the virus cannot replicate in this cell. Some viruses have developed a system to inhibit PKR and facilitate viral protein synthesis as well as viral replication. For example, adenovirus makes a large amount of a small RNA, VA1 RNA. VA1 RNA has extensive secondary structures and binds to PKR in competition with the double-stranded RNA (dsRNA) which normally activates PKR. Since it requires a minimum length of dsRNA to activate PKR, VA1 RNA does not activate PKR. Instead, it sequesters PKR by virtue of its large amount. Consequently, protein synthesis is not blocked, and adenovirus can replicate in the cell.

Ras-activated neoplastic cells are not subject to protein synthesis inhibition by PKR because ras inactivates PKR. These cells are therefore susceptible to viral infection even if the virus does not have a PKR-inhibitory system. Accordingly, if the PKR inhibitors in adenovirus, vaccinia virus, herpes simplex virus, or parapoxvirus orf virus are mutated so as not to block PKR function anymore, the resulting viruses do not infect normal cells due to protein synthesis inhibition by PKR, but they replicate in ras-activated neoplastic or cancer cells which lack PKR activities. By way of example, reoviruses selectively replicate and lyse ras-activated neoplastic or cancer cells.

Accordingly, a virus, including modified or mutated viruses, that do not inhibit PKR function, selectively replicates in ras-activated neoplastic cells while normal cells are resistant. Optionally, the oncolytic virus is an adenovirus mutated in the VA1 region, a vaccinia virus mutated in the K3L and/or E3L region, a vaccinia virus mutated in the thymidine kinase (TK) gene, a vaccinia virus mutated in the vaccinia growth factor (VGF) gene, a herpes virus mutated in the γ134.5 gene, a parapoxvirus orf virus mutated in the OV20.0L gene, or an influenza virus mutated in the NS-1 gene.

Vaccinia viruses mutated in the viral thymidine kinase (TK) gene are unable to make nucleotides needed for DNA replication. In normal cells, the cellular TK levels are usually very low and the virus is unable to replicate. In tumors, loss of the tumor suppressor Rb or an increase in cyclin activity, leads to E2F pathway activation and high levels of TK expression. Thus, cancer cells have high TK levels and the mutated vaccinia virus can replicate and spread.

The vaccinia growth factor (VGF) gene is a homolog of mammalian epidermal growth factor (EGF) and can bind and activate the EGF Receptor (EGFR). Vaccinia viruses mutated in the VGF gene are growth restricted to cells with activated EGF pathways, which is commonly mutated in cancers.

The viruses can be modified or mutated according to the known structure-function relationship of the viral PKR inhibitors. For example, since the amino terminal region of E3 protein interacts with the carboxy-terminal region domain of PKR, deletion or point mutation of this domain prevents anti-PKR function (Chang et al., PNAS 89:4825-4829 (1992); Chang, H. W. et al., Virology 194:537-547 (1993); Chang et al., J. Virol. 69:6605-6608 (1995); Sharp et al., Virol. 250:301-315 (1998); and Romano et al., Mol. and Cell. Bio. 18:7304-7316 (1998)). The K3L gene of vaccinia virus encodes pK3, a pseudosubstrate of PKR. Truncations or point mutations within the C-terminal portion of K3L protein that is homologous to residues 79 to 83 in eIF-2 abolish PKR inhibitory activity (Kawagishi-Kobayashi, M., et al., Mol. Cell. Biology 17:4146-4158 (1997)).

Another example is the Delta24 virus, which is a mutant adenovirus carrying a 24 base pair deletion in the E1A region (Fueyo, J., et al., Oncogene 19(1):2-12 (2000)). This region is responsible for binding to the cellular tumor suppressor Rb and inhibiting Rb function, thereby allowing the cellular proliferative machinery, and hence virus replication, to proceed in an uncontrolled fashion. Delta24 has a deletion in the Rb binding region and does not bind to Rb. Therefore, replication of the mutant virus is inhibited by Rb in a normal cell. However, if Rb is inactivated and the cell becomes neoplastic, Delta24 is no longer inhibited. Instead, the mutant virus replicates efficiently and lyses the Rb-deficient cell.

In addition, vesicular stomatitis virus (VSV) selectively kills neoplastic cells (and interferon can be added). A herpes simplex virus 1 (HSV-1) mutant defective in ribonucleotide reductase expression, hrR3, replicates in colon carcinoma cells but not normal liver cells (Yoon, S. S. et al., FASEB J. 14:301-311(2000)). Newcastle disease virus (NDV) replicates preferentially in malignant cells, and the most commonly used strain is 73-T (Reichard, K. W. et al., J. of Surgical Research 52:448-453 (1992); Zorn, U. et al., Cancer Biotherapy 9(3):22-235 (1994); Bar-Eli, N. et al., J. Cancer Res. Clin. Oncol. 122: 409-415 (1996)). Vaccinia virus propagates in several malignant tumor cell lines. Encephalitis virus has an oncolytic effect in a mouse sarcoma tumor, but attenuation may be required to reduce its infectivity in normal cells. Tumor regression has been described in tumor patients infected with herpes zoster, hepatitis virus, influenza, varicella, and measles virus (for a review, see Nemunaitis, J. Invest. New Drugs 17:375-386 (1999)).

Optionally, the oncolytic virus is a modified, non-reovirus virus comprising a reovirus sigma 1 protein, wherein the reovirus sigma 1 protein replaces the native attachment protein of the non-reovirus virus, and wherein the modified virus does not comprise any portion of the native attachment protein of the non-reovirus virus. In the modified, non-reovirus virus, the reovirus sigma 1 protein attaches to carrier cells that protect the virus from neutralizing antibodies during *in vivo* delivery to a tumor, for example, during systemic delivery. The non-reovirus virus can be, but is not limited to, an adenovirus, a vaccinia virus, a herpes simplex virus, a Sindbis virus, or a parapox virus. The modified, non-reovirus virus can be an oncolytic virus. Such non-reovirus viruses can be made by replacing the native attachment protein of the non-reovirus virus with a reovirus sigma 1 protein, wherein the full-length sequence of the native attachment protein of the non-reovirus virus is replaced with a reovirus sigma 1 protein. In the methods of making a non-reovirus virus, replacement of the native attachment protein of the virus with a reovirus sigma 1 protein allows the virus to attach to carrier cells which protect the virus from neutralizing antibodies during *in vivo* delivery. The reovirus sigma-1 protein is described in, for example, WO 2008/11004.

Optionally, the oncolytic virus is a reovirus. Reovirus refers to any virus classified in the reovirus genus, whether naturally occurring, modified, or recombinant. Reoviruses are viruses with a double-stranded, segmented RNA genome. The virions measure 60-80 nm in diameter and possess two concentric capsid shells, each of which is icosahedral. The genome consists of double-stranded RNA in 10-12 discrete segments with a total genome size of 16-27 kbp. The individual RNA segments vary in size. Three distinct but related types of reoviruses have been recovered from many species. Thus, the reovirus can be a mammalian reovirus or a human reovirus. All three types share a common complement-fixing antigen.

The human reovirus includes three serotypes: type 1 (strain Lang or TIL), type 2 (strain Jones, T2J), and type 3 (strain Dearing or strain Abney, T3D). The three serotypes are easily identifiable on the basis of neutralization and hemagglutinin-inhibition assays. A reovirus according to this disclosure can be a type 3 mammalian orthoreovirus. Type 3 mammalian orthoreoviruses include, without limitation, Dearing and Abney strains (T3D or T3A, respectively). See, for example, ATCC Accession Nos. VR-232 and VR-824. As described previously, reoviruses use a host cell's ras pathway machinery to downregulate double-stranded RNA-activated protein kinase (PKR) and thus replication in the cell. See, for example, U.S. Patent Nos. 6,110,461; 6,136,307; 6,261,555; 6,344,195; 6,576,234; and 6,811,775.

The reovirus may be naturally occurring or modified. The reovirus is naturally-occurring when it can be isolated from a source in nature and has not been intentionally modified by humans in the laboratory. For example, the reovirus can be from a field source, that is, from a human who has been infected with the reovirus. The reovirus may also be selected or mutagenized for enhanced oncolytic activity.

The reovirus may be modified but still capable of lytically infecting a mammalian cell having an active ras pathway. The reovirus may be chemically or biochemically pretreated (e.g., by treatment with a protease, such as chymotrypsin or trypsin) prior to administration to the proliferating cells. Pretreatment with a protease removes the outer coat or capsid of the virus and may increase the infectivity of the virus. The reovirus may be coated in a liposome or micelle (Chandran and Nibert, J. of Virology 72(1):467-75 1998). For example, the virion may be treated with chymotrypsin in the presence of micelle-forming concentrations of alkyl sulfate detergents to generate a new infectious subviral particle (ISVP).

The reovirus may be a recombinant reovirus. For example, the recombinant reovirus can be a reassortant reovirus, which includes genomic segments from two or more genetically distinct reoviruses. Recombination/reassortment of reovirus genomic segments may occur following infection of a host organism with at least two genetically distinct reoviruses. Recombinant/reassortant viruses can also be generated in cell culture, for example, by co-infection of permissive host cells with genetically distinct reoviruses. Accordingly, the provided methods include the use of a recombinant reovirus resulting from reassortment of genome segments from two or more genetically distinct reoviruses, including but not limited to, human reovirus, such as type 1 (e.g., strain Lang), type 2 (e.g., strain Jones), and type 3 (e.g., strain Dearing or strain Abney); non-human mammalian reoviruses; or avian reovirus. Optionally, the provided methods include the use of recombinant reoviruses resulting from reassortment of genome segments from two or more genetically distinct reoviruses wherein at least one parental virus is genetically engineered, comprises one or more chemically synthesized genomic segment, has been treated with chemical or physical mutagens, or is itself the result of a recombination event. Optionally, the provided methods include the use of the recombinant reovirus that has undergone recombination in the presence of chemical mutagens, including but not limited to, dimethyl sulfate and ethidium bromide, or physical mutagens, including but not limited to, ultraviolet light and other forms of radiation.

Optionally, the provided methods include the use of reoviruses with mutations (including insertions, substitutions, deletions or duplications) in one or more genome segments. Such mutations can comprise additional genetic information as a result of recombination with a host cell genome or can comprise synthetic genes. For example, mutant reoviruses as described herein can contain a mutation that reduces or essentially eliminates expression of a sigma3 polypeptide or that results in the absence of a functional sigma3 polypeptide as described in U.S. Publication No. 2008/0292594. A mutation that eliminates expression of a sigma3 polypeptide or that results in the absence of a functional sigma3 polypeptide can be in the nucleic acid encoding the sigma3 polypeptide (i.e., the S4 gene) or in a nucleic acid that encodes a polypeptide that regulates the expression or function of the sigma3 polypeptide.

As used herein, a mutation that reduces the expression of a sigma3 polypeptide refers to a mutation that results in a decrease in the amount of sigma3 polypeptides, compared to a reovirus expressing wild type levels of sigma3 polypeptide, of at least 30% (e.g., at least 40%, 50%, 60%, 70%, 80%, 90%, or 95%). As used herein, a mutation that essentially eliminates expression of a sigma3 polypeptide refers to a mutation that results in a decrease in the amount of sigma3 polypeptides, relative to the amount of sigma3 polypeptides produced by a wild type reovirus, of at least 95% (e.g., 96%, 97%, 98%, 99%, or 100%). As used herein, a mutation that results in a decrease in or absence of a functional sigma3 polypeptide refers to a mutation that allows expression of the sigma3 polypeptide but that results in a sigma3 polypeptide that is not able to assemble or incorporate into the viral capsid. It would be understood that it may be desirable or necessary for sigma3 polypeptides to retain other functionalities (e.g., the ability to bind RNA) in order that the mutant reovirus retain the ability to propagate.

A mutation in a sigma3 polypeptide as described herein can result in a sigma3 polypeptide that is incorporated into the capsid at levels that are reduced relative to a sigma3 polypeptide that does not contain the mutation (e.g., a wild type sigma3 polypeptide). A mutation in a sigma3 polypeptide as described herein also can result in a sigma3 polypeptide that cannot be incorporated into a viral capsid. Without being bound by any particular mechanism, a sigma3 polypeptide may have reduced function or lack function due, for example, to an inability of the sigma3 polypeptide and the mu1 polypeptide to bind appropriately, or due to a conformational change that reduces or prohibits incorporation of the sigma3 polypeptide into the capsid.

In addition to a mutation that abolishes or reduces expression of the sigma3 polypeptide or that results in a non-functional or reduced-function sigma3 polypeptide, a mutant reovirus as described herein also can contain one or more further mutations (e.g., a second, third, or fourth mutation) in one of the other reovirus capsid polypeptides (e.g., mu1, lambda2, and/or sigmal). Reoviruses containing a mutation affecting the sigma3 polypeptide and, optionally, a further mutation in any or all of the other outer capsid proteins can be screened for the ability of such mutant reoviruses to infect and cause lysis of cells. For example, neoplastic cells that are resistant to lysis by wild type reovirus can be used to screen for effective mutant reoviruses described herein.

For example, a further mutation can reduce or essentially eliminate expression of a mu1 polypeptide or result in the absence of a functional mu1 polypeptide. The mu1 polypeptide, which is encoded by the M2 gene, is likely involved in cell penetration and may play a role in transcriptase activation. Each virion contains about 600 copies of mu1 polypeptides, which are present in the form of 1:1 complexes with sigma3 polypeptides. The mu1 polypeptide is myristolated on its N-terminus, and then the myristolated N-terminal 42 residues are cleaved off, resulting in a C-terminal fragment (mu1C). Additionally or alternatively, a further mutation can reduce or essentially eliminate expression of a lambda2 polypeptide or result in the absence of a functional lambda2 polypeptide, and/or a further mutation can reduce or essentially eliminate expression of a sigma1 polypeptide or result in the absence of a functional sigma1 polypeptide. The lambda2 polypeptide is encoded by the L2 gene, is involved in particle assembly, and exhibits guanylyltransferase and methyltransferase activity. The sigma1 polypeptide is encoded by the S1 gene, is involved in cell-attachment and serves as the viral hemagglutinin.

Optionally, the reovirus comprises a lambda-3 polypeptide having one or more amino acid modifications, a sigma-3 polypeptide having one or more amino acid modifications, a mu-1 polypeptide having one or more amino acid modifications, a mu-2 polypeptide having one or more amino acid modifications, or any combination thereof. For example, the reovirus has a lambda-3 polypeptide having one or more amino acid modifications; a sigma-3 polypeptide having one or more amino acid modifications; a mu-1 polypeptide having one or more amino acid modifications; and/or a mu-2 polypeptide having one or more amino acid modifications, as described in U.S. Serial No. 12/046,095. By way of example, the one or more amino acid modifications in the lambda-3 polypeptide are a Val at residue 214, an Ala at residue 267, a Thr at residue 557, a Lys at residue 755, a Met at residue 756, a Pro at residue 926, a Pro at residue 963, a Leu at residue 979, an Arg at residue 1045, a Val at residue 1071, or any combination thereof, numbered relative to GenBank Accession No. M24734.1 (SEQ ID NO:23). It is noted that, when the amino acid sequence is a Val at residue 214 or a Val at residue 1071, the amino acid sequence further includes at least one additional change in the amino acid sequence. Optionally, the lambda-3 polypeptide includes the sequence shown in SEQ ID NO:19. Further by way of example, the one or more amino acid modifications in the sigma-3 polypeptide are a Leu at residue 14, a Lys at residue 198, or any combination thereof, numbered relative to GenBank Accession No. K02739 (SEQ ID NO:25). It is noted that, when the amino acid sequence is a Leu at residue 14, the amino acid sequence further includes at least one additional change in the amino acid sequence. Optionally, the sigma-3 polypeptide includes the sequence shown in SEQ ID NO:15. Further by way of example, the one or more amino acid modifications in the mu-1 polypeptide is an Asp at residue 73 numbered relative to GenBank Accession No. M20161.1 (SEQ ID NO:27). Optionally, the mu-1 polypeptide includes the sequence shown in SEQ ID NO:17. Also by way of example, the amino acid modification mu-2 polypeptide is a Ser at residue 528 numbered relative to GenBank Accession No. AF461684.1 (SEQ ID NO:29). Optionally, the mu-1 polypeptide includes the sequence shown in SEQ ID NO:17. A reovirus as described herein having one or more modifications can further include a reovirus sigma-2 polypeptide. Such a sigma-2 polypeptide has a Cys at one or more of position 70, 127, 195, 241, 255, 294, 296, or 340, numbered relative to GenBank Accession No. NP_694684.1 (SEQ ID NO:30). Optionally, the sigma-2 polypeptide includes the sequence shown in SEQ ID NO:12.

Optionally, the reovirus comprises a L1 genome segment comprising one or more nucleic acid modifications, an S4 genome segment comprising one or more nucleic acid modifications, an M1 genome segment comprising one or more nucleic acid modifications, an M2 genome segment comprising one or more nucleic acid modifications, or any combination thereof. Optionally, the reovirus has a L1 genome segment having one or more nucleic acid modifications; a S4 genome segment having one or more nucleic acid modifications; a M1 genome segment having one or more nucleic acid modifications; and/or a M2 genome segment having one or more nucleic acid modifications, as described in WO 2008/110004. By way of example, the one or more nucleic acid modifications in the L1 genome segment are a T at position 660, a G at position 817, an A at position 1687, a G at position 2283, an ATG at positions 2284-2286, a C at position 2794, a C at position 2905, a C at position 2953, an A at position 3153, or a G at position 3231, numbered relative to GenBank Accession No. M24734.1 (SEQ ID NO:22). Optionally, the L1 genome segment includes the sequence shown in SEQ ID NO:8. Further by way of example, the one or more nucleic acid modifications in the S4 genome segment is an A at position 74 and an A at position 624, numbered relative to GenBank Accession No. K02739 (SEQ ID NO:24). Optionally, the S4 genome segment includes the sequence shown in SEQ ID NO:4. Further by way of example, the nucleic acid modification in the M2 genome segment can be a C at position 248, numbered relative to GenBank Accession No. M20161.1 (SEQ ID NO:26). The M2 genome segment, for example, includes the sequence shown in SEQ ID NO:6. Also by way of example, the nucleic acid modification in the M1 genome segment is a T at position 1595, numbered relative to GenBank Accession No. AF461684.1 (SEQ ID NO:28). Optionally, the M1 genome segment includes the sequence shown in SEQ ID NO:5. A reovirus as described herein can include any modification or combination of modifications disclosed herein. Optionally, a reovirus as described herein includes genomic segments having the sequences shown in SEQ ID NOs:1-10 or the polypeptides shown in SEQ ID NOs:11, 12, and 16-21, and either or both SEQ ID NO:13 or 14. Optionally, a reovirus as disclosed herein is identified as IDAC Accession No. 190907-01, which was deposited with the International Depositary of Canada (IDAC, National Microbiology Laboratory, Public Health Agency of Canada, 1015 Arlington St., Winnipeg, Manitoba Canada R3E 3R2 on September 19, 2007. The appended claims specify that the oncolytic virus is a reovirus having IDAC Accession No. 190907-01.

Sindbis virus (SIN) can be used in the methods described herein. Sindbis virus is a member of the alphavirus genus of the togaviridae family. The Sindbis virus genome is a single-stranded RNA of 11703 nucleotides, capped at the 5' terminus and poly-adenylated at the 3' terminus. The genome consists of a 49S untranslated region (UT), nonstructural proteins nsP1, nsP2, nsP3, and nsP4 followed by a promoter. The promoter is followed by a 26S UT, structural proteins C, E3, E2, 6K, and E1 and finally a 3' UT and a poly-adenylated terminus. The genomic 49S RNA is of plus sense, is infectious, and serves as mRNA in the infected cell.

Sindbis vectors systemically and specifically infect/detect and kill metastasized tumors *in vivo,* leading to significant suppression of tumor growth and enhanced survival (Hurtado et al., Rejuvenation Res. 9(1):36-44 (2006)). Sindbis virus infects mammalian cells using the Mr 67,000 laminin receptor, which is elevated in tumor versus normal cells. Tumor overexpression of the laminin receptor may explain the specificity and efficacy that Sindbis vectors demonstrate for tumor cells *in vivo.* Sindbis does not have to undergo genetic manipulation to target cancer cells or to be injected directly into tumors. Sindbis injected anywhere into a subject travels through the bloodstream to the target area (Tseng et al., Cancer Res. 64(18):6684-92 (2004). Sindbis can also be genetically engineered to carry one or more genes that suppress the immune response to the virus and/or genes that stimulate an immune response against the tumor such as, for example, antitumor cytokine genes such as interleukin-12 and interleukin-15 genes.

The oncolytic virus may be naturally occurring or modified. The virus may be chemically or biochemically pretreated (e.g., by treatment with a protease, such as chymotrypsin or trypsin) prior to administration to the neoplastic cells. Pretreatment with a protease removes the outer coat or capsid of the virus and may increase the infectivity of the virus. The virus may be coated in a liposome or micelle (Chandran and Nibert, J. of Virology 72(1):467-75 (1998)) to reduce or prevent an immune response from a mammal which has developed immunity to the virus. For example, the virion may be treated with chymotrypsin in the presence of micelle forming concentrations of alkyl sulfate detergents to generate a new infectious subvirion particle. The oncolytic virus may also be a reassortant virus or an ISVP.

The present methods include using any oncolytic virus according to the disclosure herein and knowledge available in the art. The oncolytic virus may be naturally occurring or modified. The oncolytic virus is naturally-occurring when it can be isolated from a source in nature and has not been intentionally modified by humans in the laboratory. For example, the oncolytic virus can be from a field source, that is, from a human who has been infected with the oncolytic virus.

The oncolytic virus may be a recombinant oncolytic virus. For example, the recombinant oncolytic virus results from the reassortment of genomic segments from two or more genetically distinct oncolytic viruses, also referred to herein as a reassortant. Reassortment of oncolytic virus genomic segments may occur following infection of a host organism with at least two genetically distinct oncolytic viruses. Recombinant viruses can also be generated in cell culture, for example, by co-infection of permissive host cells with genetically distinct oncolytic viruses. Optionally, the methods include the use of recombinant oncolytic virus resulting from reassortment of genome segments from two or more genetically distinct oncolytic viruses wherein at least one parental virus is genetically engineered, comprises one or more chemically synthesized genomic segment, has been treated with chemical or physical mutagens, or is itself the result of a recombination event. Optionally, the methods include the use of the recombinant oncolytic virus that has undergone recombination in the presence of chemical mutagens, including but not limited to dimethyl sulfate and ethidium bromide, or physical mutagens, including but not limited to ultraviolet light and other forms of radiation.

Optionally, the methods include the use of oncolytic viruses with mutations including (insertions, substitutions, deletions or duplications) in one or more genome segments. Such mutations can comprise additional genetic information as a result of recombination with a host cell genome, or that comprise synthetic genes such as, for example, genes encoding agents that suppress anti-viral immune responses.

Optionally, the oncolytic virus is a mutant oncolytic virus. For example, the oncolytic virus may be modified by incorporation of mutated coat proteins, such as for example, into the virion outer capsid. The mutant oncolytic virus is, optionally, a mutant reovirus. Mutant reoviruses as described herein can contain a mutation that reduces or essentially eliminates expression of a sigma3 polypeptide or that results in the absence of a functional sigma3 polypeptide as described in U.S. Serial No. 12/124,522. Optionally, the mutant reoviruses used in the provided methods are mutated as described in U.S. Serial No. 12/046,095.

A mutation as referred to herein can be a substitution, insertion or deletion of one or more nucleotides. Point mutations include, for example, single nucleotide transitions (purine to purine or pyrimidine to pyrimidine) or transversions (purine to pyrimidine or vice versa) and single- or multiple-nucleotide deletions or insertions. A mutation in a nucleic acid can result in one or more conservative or non-conservative amino acid substitutions in the encoded polypeptide, which may result in conformational changes or loss or partial loss of function, a shift in the reading frame of translation (frame-shift) resulting in an entirely different polypeptide encoded from that point on, a premature stop codon resulting in a truncated polypeptide (truncation), or a mutation in a virus nucleic acid may not change the encoded polypeptide at all (silent or nonsense). See, for example, Johnson and Overington, 1993, J. Mol. Biol. 233:716-38; Henikoff and Henikoff, 1992, Proc. Natl. Acad. Sci. USA 89:10915-19; and U.S. Patent No. 4,554,101, for disclosure on conservative and non-conservative amino acid substitutions.

Mutations can be generated in the nucleic acid of an oncolytic virus using any number of methods known in the art. For example, site directed mutagenesis can be used to modify a reovirus nucleic acid sequence. One of the most common methods of site-directed mutagenesis is oligonucleotide-directed mutagenesis. In oligonucleotide-directed mutagenesis, an oligonucleotide encoding the desired change(s) in sequence is annealed to one strand of the DNA of interest and serves as a primer for initiation of DNA synthesis. In this manner, the oligonucleotide containing the sequence change is incorporated into the newly synthesized strand. See, for example, Kunkel, 1985, Proc. Natl. Acad. Sci. USA 82:488; Kunkel et al., 1987, Meth. Enzymol. 154:367; Lewis and Thompson, 1990, Nucl. Acids Res. 18:3439; Bohnsack, 1996, Meth. Mol. Biol. 57:1; Deng and Nickoloff, 1992, Anal. Biochem. 200:81; and Shimada, 1996, Meth. Mol. Biol. 57:157. Other methods are used routinely in the art to modify the sequence of a protein or polypeptide. For example, nucleic acids containing a mutation can be generated using PCR or chemical synthesis, or polypeptides having the desired change in amino acid sequence can be chemically synthesized. See, for example, Bang and Kent, 2005, Proc. Natl. Acad. Sci. USA 102:5014-9 and references therein.

Viruses can be purified using standard methodology. See, for example, Schiff et al., "Orthoreoviruses and Their Replication," Ch 52, in Fields Virology, Knipe and Howley, eds., 2006, Lippincott Williams and Wilkins; Smith et al., 1969, Virology 39(4):791-810; and U.S. Patent Nos. 7,186,542; 7,049,127; 6,808,916; and 6,528,305. As used herein, purified viruses refer to viruses that have been separated from cellular components that naturally accompany them. Typically, viruses are considered purified when they are at least 70% (e.g., at least 75%, 80%, 85%, 90%, 95%, or 99%) by dry weight, free from the proteins and other cellular components with which they are naturally associated.

As discussed above, chemotherapeutic agents include, but are not limited to, alkylating agents, anthracyclines, taxanes, epothilones, histone deacetylase inhibitors, inhibitors of Topoisomerase I, inhibitors of Topoisomerase II, kinase inhibitors, monoclonal antibodies, nucleotide analogs and precursor analogs, peptide antibiotics, platinum-based compounds, retinoids, and vinca alkaloids and derivatives.

Examples of alkylating agents include, but are not limited to, nitrogen mustards, nitrosoureas, tetrazines, aziridines, cisplatins and derivatives, and non-classical alkylating agents. Specific examples of alkylating agents include, but are not limited to, mechlorethamine, cyclophosphamide, melphalan, chlorambucil, ifosfamide, busulfan, N-Nitroso-N-methylurea (MNU), carmustine (BCNU), lomustine (CCNU), semustine (MeCCNU), fotemustine, streptozotocin, dacarbazine, mitozolomide, temozolomide, thiotepa, mytomycin, diaziquone, cisplatin, carboplatin, oxaliplatin, procarbazine and hexamethylmelamine.

Other exemplary chemotherapeutic agents include but are not limited to 5-fluorouracil, mitomycin C, methotrexate, hydroxyurea, mitoxantrone, anthracyclins (e.g., epirubicin and doxurubicin), antibodies to receptors (e.g., herceptin, etoposide, pregnasome), hormone therapies (e.g., tamoxifen and anti-estrogens), interferons, aromatase inhibitors, progestational agents, and LHRH analogs. Optionally, the chemotherapeutic agent is a platinum compound. Optionally, the platinum compound is selected from the group consisting of cisplatin, carboplatin, oxaliplatin, satraplatin, picoplatin, nedaplatin, and triplatin. Optionally, the chemotherapeutic agent is a taxane. Optionally, the taxane is selected from the group consisting of cabazitaxel, abraxane, paclitaxel and docetaxel.

As discussed throughout, chemotherapy and administration of a chemotherapeutic agent can include administration of more than one chemotherapeutic agent to the subject, i.e., administration of a combination of chemotherapeutic agents. Thus, for example, the chemotherapeutic agent is a combination selected from the group consisting of (i) an alkylating agent and an antibiotic, (ii) an antibiotic and a platinum compound, (iii) an alkylating agent and a nucleotide analog, (iv) a platinum compound and a taxane, (v) a platinum compound, a taxane and an antibody. Optionally, the chemotherapeutic agent is a platinum compound and a taxane. Optionally, the chemotherapeutic agent is carboplatin and paclitaxel.

Provided herein are pharmaceutical compositions comprising one or more oncolytic viruses. Also provided are pharmaceutical compositions comprising one or more chemotherapeutic agents. Optionally, the pharmaceutical compositions include one or more oncolytic viruses and one or more chemotherapeutic agents. Thus, the provided compositions can include a single agent or more than one agent.

The herein provided compositions are administered *in vitro* or *in vivo* in a pharmaceutically acceptable carrier. A pharmaceutically acceptable carrier can be a solid, semisolid, or liquid material that can act as a vehicle, carrier or medium for the reovirus. Thus, compositions containing an oncolytic virus and/or one or more of the provided agents can be in the form of tablets, pills, powders, lozenges, sachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

Optionally, the compositions containing an oncolytic virus are suitable for infusion. For intravenous infusions, there are two types of fluids that are commonly used, crystalloids and colloids. Crystalloids are aqueous solutions of mineral salts or other water-soluble molecules. Colloids contain larger insoluble molecules, such as gelatin; blood itself is a colloid. The most commonly used crystalloid fluid is normal saline, a solution of sodium chloride at 0.9% concentration, which is close to the concentration in the blood (isotonic). Ringer's lactate or Ringer's acetate is another isotonic solution often used for large-volume fluid replacement. A solution of 5% dextrose in water, sometimes called D5W, is often used instead if the patient is at risk for having low blood sugar or high sodium.

Some examples of suitable carriers include phosphate-buffered saline or another physiologically acceptable buffer, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. A pharmaceutical composition additionally can include, without limitation, lubricating agents such as talc, magnesium stearate, and mineral oil; wetting agents; emulsifying and suspending agents; preserving agents such as methyl- and propylhydroxy-benzoates; sweetening agents; and flavoring agents. Pharmaceutical compositions can be formulated to provide quick, sustained or delayed release of a mutant reovirus after administration by employing procedures known in the art. In addition to the representative formulations described below, other suitable formulations for use in a pharmaceutical composition can be found in Remington: The Science and Practice of Pharmacy 22d edition Loyd V. Allen et al, editors, Pharmaceutical Press (2012). For preparing solid compositions such as tablets, a mutant reovirus can be mixed with a pharmaceutical carrier to form a solid composition. Optionally, tablets or pills can be coated or otherwise compounded to provide a dosage form affording the advantage of prolonged action. For example, a tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Liquid formulations that include a reovirus and/or agent for oral administration or for injection generally include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. These liquid or solid compositions may contain suitable pharmaceutically acceptable excipients as described herein. Such compositions can be administered by the oral or nasal respiratory route for local or systemic effect. Compositions in pharmaceutically acceptable solvents may be nebulized by use of inert gases. Nebulized solutions may be inhaled directly from the nebulizing device or the nebulizing device may be attached to a face mask tent or intermittent positive pressure breathing machine. Solution, suspension, or powder compositions may be administered, orally or nasally, from devices which deliver the formulation in an appropriate manner.

Another formulation that is optionally employed in the methods of the present disclosure includes transdermal delivery devices (e.g., patches). Such transdermal patches may be used to provide continuous or discontinuous infusion of the viruses and agents as described herein. The construction and use of transdermal patches for the delivery of pharmaceutical agents is well known in the art. See, for example, U.S. Patent No. 5,023,252. Such patches can be constructed for continuous, pulsatile, or on-demand delivery of mutant reoviruses.

As described above, viruses and/or other agents can, if necessary, be coated in a liposome or micelle to reduce or prevent an immune response in a mammal that has developed immunity toward a virus or agent. Such compositions are referred to as immunoprotected viruses or agents. See, for example, U.S. Patent Nos. 6,565,831 and 7,014,847.

In the provided methods, the oncolytic virus is administered, for example, systemically, in a manner so that it can ultimately contact the target tumor or tumor cells. The route by which the virus is administered, as well as the formulation, carrier or vehicle, depends on the location as well as the type of the target cells. A wide variety of administration routes can be employed. For example, for a solid tumor that is accessible, the virus can be administered by injection directly to the tumor. For a hematopoietic tumor, for example, the virus can be administered intravenously or intravascularly. For tumors that are not easily accessible within the body, such as metastases, the virus is administered in a manner such that it can be transported systemically through the body of the mammal and thereby reach the tumor (e.g., intravenously or intramuscularly). Alternatively, the virus can be administered directly to a single solid tumor, where it then is carried systemically through the body to metastases. The virus can also be administered subcutaneously, intraperitoneally, intrathecally or intraventricularly (e.g., for brain tumor), topically (e.g., for melanoma), orally (e.g., for oral or esophageal cancer), rectally (e.g., for colorectal cancer), vaginally (e.g., for cervical or vaginal cancer), nasally, by inhalation spray or by aerosol formulation (e.g., for lung cancer).

Optionally, the virus is administered continuously to a subject at least once per day or up to intermittently or continuously throughout the day on consecutive days, for a period of time. Thus, the virus is administered, for example, to subjects by means of intravenous administration in any pharmacologically acceptable solution, or as an infusion over a period of time. For example, the substance may be administered systemically by injection (e.g., IM or subcutaneously) or taken orally daily at least once per day, or administered by infusion in a manner that results in the daily delivery into the tissue or blood stream of the subject. When the virus is administered by infusion over a period of time, the period of time is, for example, 1, 2, 3, 4, 5, 6, 7, 8, 9, 10, 12, or 24 hours, or any time between 1 and 24 hours, inclusive, or more. Optionally, the period of time is 5, 15, 30, 60, 90, 120, 150 or 180 minutes, or any time between 5 and 180 minutes, inclusive, or more. Thus, for example, the virus is administered by infusion for 60 minutes. Administrations can be repeated daily for 2, 3, 4, 5, 6, 7, 8, 9, 10, 14, 21, 28 days or any number of days between 2 and 28 days, inclusive, or longer.

The viruses as disclosed herein are administered in an amount that is sufficient (i.e., an effective amount) to effect the treatment of the cancer or proliferative disorder. A proliferative disorder is treated when administration of a treatment regimen including a virus to proliferating cells affects lysis (e.g., oncolysis) of the affected cells, resulting in a reduction in the number of abnormally, proliferating cells, a reduction in the size of a neoplasm, and/or a reduction in or elimination of symptoms (e.g., pain) associated with the proliferating disorder. As used herein, the term oncolysis means at least 10% of the proliferating cells are lysed (e.g., at least about 20%, 30%, 40%, 50%, or 75% of the cells are lysed). The percentage of lysis can be determined, for example, by measuring the reduction in the size of a neoplasm or in the number of proliferating cells in a mammal, or by measuring the amount of lysis of cells *in vitro* (e.g., from a biopsy of the proliferating cells). An effective amount of a virus used in a treatment regimen will be determined on an individual basis and may be based, at least in part, on the particular virus used; the individual's size, age, gender; and the size and other characteristics of the abnormally, proliferating cells. For example, for treatment of a human, approximately 10³ to 10¹² plaque forming units (PFU) of a virus are used, depending on the type, size and number of proliferating cells or neoplasms present. The effective amount can be, for example, from about 1.0 PFU/kg body weight to about 10¹⁵ PFU/kg body weight (e.g., from about 10² PFU/kg body weight to about 10¹³ PFU/kg body weight). Optionally, the effective amount is about 1x10⁸ to about 1x10¹² PFU or TCID₅₀. Optionally, the effective amount is about 3x10¹⁰ to about 1x10¹⁰ TCID₅₀.

Optimal dosages of viruses and therapeutic agents, and compositions and kits comprising viruses and agents depend on a variety of factors. The exact amount required will vary from subject to subject, depending on the species, age, weight and general condition of the subject, the severity of the disease being treated, the particular virus or vector used and its mode of administration. Thus, it is not possible to specify an exact amount for every composition or kit. However, an appropriate amount can be determined by one of ordinary skill in the art using only routine experimentation given the guidance provided herein.

Effective dosages and schedules for administering the treatment regimens may be determined empirically. For example, animal models for a variety of proliferative disorders can be obtained from the Jackson Laboratory, 600 Main Street, Bar Harbor, Maine 04609 USA. Both direct (e.g., histology of tumors) and functional measurements (e.g., survival of a subject or size of a tumor) can be used to monitor response to therapies. These methods involve the sacrifice of representative animals to evaluate the population, increasing the animal numbers necessary for the experiments. Measurement of luciferase activity in the tumor provides an alternative method to evaluate tumor volume without animal sacrifice and allowing longitudinal population-based analysis of therapy.

The dosage ranges for the administration of compositions are those large enough to produce the desired effect in which the symptoms of the disease are affected. The dosage should not be so large as to cause adverse side effects, such as unwanted cross-reactions and anaphylactic reactions. The dosage can be adjusted by the individual physician in the event of any counterindications.

Dosages vary and are administered in one or more dose administrations, for example, daily, for one or several days. The provided viruses and therapeutic agents are administered in a single dose or in multiple doses (e.g., two, three, four, six, or more doses). For example, where the administration is by infusion, the infusion can be a single sustained dose or can be delivered by multiple infusions. Treatment may last from several days to several months or until diminution of the disease is achieved.

The provided methods may be further combined with other tumor therapies such as radiotherapy, surgery, hormone therapy and/or immunotherapy. Thus, the provided methods can further include administering one or more additional therapeutic agents to the subject. Suitable additional therapeutic agents include, but are not limited to, analgesics, anesthetics, analeptics, corticosteroids, anticholinergic agents, anticholinesterases, anticonvulsants, antineoplastic agents, allosteric inhibitors, anabolic steroids, antirheumatic agents, psychotherapeutic agents, neural blocking agents, anti-inflammatory agents, antihelmintics, antibiotics, anticoagulants, antifungals, antihistamines, antimuscarinic agents, antimycobacterial agents, antiprotozoal agents, antiviral agents, dopaminergics, hematological agents, immunological agents, muscarinics, protease inhibitors, vitamins, growth factors, and hormones. The choice of agent and dosage can be determined readily by one of skill in the art based on the given disease being treated.

Combinations of the provided viruses and therapeutic agents are administered either concomitantly (e.g., as an admixture), separately but simultaneously (e.g., via separate intravenous lines into the same subject), or sequentially (e.g., one of the compounds or agents is given first followed by the second). Thus, the term combination is used to refer to concomitant, simultaneous, or sequential administration of two or more agents.

When one compound is administered prior to another compound, the first compound is administered minutes, hours, days, or weeks prior to administration of the second compound. For example, the first compound can be administered at 1, 2, 3, 4, 5, 6, 7, 8, 9, 10, 12, 24, 36, 48, 60, or 72 hours, or any time between 1 and 72 hours, inclusive, prior to administration of a second compound. Optionally, the first compound is administered more than 72 hours prior to the second compound. By way of another example, the first compound can be administered at 1, 5, 15, 30, 60, 90, 120, 150 or 180 minutes, or any time between 1 and 180 minutes, inclusive, prior to administration of a second compound. Optionally, the first compound is administered at 1, 2, 3, 4, 5, 6, 7, 14, 21, or 28 days, or any amount in between 1 and 28, inclusive, days prior to administration of the second compound. Optionally, the first compound is administered more than 28 days prior to the second compound.

Oncolytic viruses or a pharmaceutical composition comprising such viruses can be packaged into a kit. The kit also includes one or more chemotherapeutic agents or pharmaceutical compositions comprising the chemotherapeutic agents. Thus, provided herein are kits comprising an oncolytic virus and a chemotherapeutic agent, wherein the chemotherapeutic agent comprises an amount for an increased treatment regimen of a chemotherapeutic agent as compared to standard therapy. The increased treatment regimen can include an increased amount per dose of chemotherapeutic agent as compared to standard therapy or an increased number of doses of the chemotherapeutic agent as compared to standard therapy. Optionally, the increased treatment regimen comprises an increased amount per dose and an increased number of doses of chemotherapeutic agent as compared to standard therapy. The increased treatment regimen can include an increased number of treatment cycles with the chemotherapeutic agent as compared to standard therapy. Optionally, the increased number of treatment cycles comprises more than four treatment cycles with the chemotherapeutic agent. Optionally, the increased number of treatment cycles comprises 5, 6, 7, 8, 9, 10, 11, 12, 13, 14, 15, 16, 17, 18, 19, 20, 21, 22, 23, 24, 25 or more treatment cycles with the chemotherapeutic agent.

The oncolytic viruses and/or chemotherapeutic agents and pharmaceutical compositions containing the same can be packaged in one or more containers. When the kits contain pharmaceutical compositions, the pharmaceutical compositions can be formulated in a unit dosage form. The term "unit dosage forms" refers to physically discrete units suitable as unitary dosages for human subjects and other mammals, each unit containing a predetermined quantity of an oncolytic virus or other agent, e.g., chemotherapeutic agent calculated to produce the desired therapeutic effect in association with a suitable pharmaceutically acceptable carrier.

Optionally, the kit includes one or more chemotherapeutic agents or a combination of chemotherapeutic agents. Chemotherapeutic agents include, but are not limited to, alkylating agents, anthracyclines, taxanes, epothilones, histone deacetylase inhibitors, inhibitors of Topoisomerase I, inhibitors of Topoisomerase II, kinase inhibitors, monoclonal antibodies, nucleotide analogs and precursor analogs, peptide antibiotics, platinum-based compounds, retinoids, and vinca alkaloids and derivatives. Exemplary chemotherapeutic agents include but are not limited to 5-fluorouracil, mitomycin C, methotrexate, hydroxyurea, mitoxantrone, anthracyclins (e.g., epirubicin and doxurubicin), antibodies to receptors (e.g., herceptin, etoposide, pregnasome), hormone therapies (e.g., tamoxifen and anti-estrogens), interferons, aromatase inhibitors, progestational agents, and LHRH analogs. Optionally, the chemotherapeutic agent is a platinum compound. Optionally, the platinum compound is selected from the group consisting of cisplatin, carboplatin, oxaliplatin, satraplatin, picoplatin, nedaplatin, and triplatin. Optionally, the chemotherapeutic agent is a taxane. Optionally, the taxane is selected from the group consisting of cabazitaxel, abraxane, paclitaxel and docetaxel. Optionally, the kit includes a combination of chemotherapeutic agents selected from the group consisting of (i) an alkylating agent and an antibiotic, (ii) an antibiotic and a platinum compound, (iii) an alkylating agent and a nucleotide analog, (iv) a platinum compound and a taxane, (v) a platinum compound, a taxane and an antibody. Optionally, the chemotherapeutic agent is a platinum compound and a taxane. Optionally, the chemotherapeutic agent is carboplatin and paclitaxel.

The oncolytic virus in the provided kits can be any of the oncolytic viruses described herein. Optionally, the oncolytic virus is selected from the group consisting of a reovirus, a Newcastle disease virus (NDV), a vesicular stomatitis virus (VSV), an adenovirus, a vaccinia virus, a parapox orf virus, a Sindbis virus, and a herpes simplex virus. Optionally, the reovirus is a mammalian reovirus, a human reovirus, a serotype 1 reovirus, a serotype 2 reovirus, a serotype 3 reovirus, or a Dearing strain reovirus. Optionally, as discussed above, the human reovirus has IDAC Accession No. 190907-01. The reovirus can include a lambda-3 polypeptide having one or more amino acid modifications, a sigma-3 polypeptide having one or more amino acid modifications, a mu-1 polypeptide having one or more amino acid modifications, a mu-2 polypeptide having one or more amino acid modifications, or any combination thereof. Optionally, the reovirus comprises one or more of a sigma-3 polypeptide having one or more amino acid modifications, wherein the one or more amino acid modifications are selected from the group consisting of a Leu at residue 14, a Lys at residue 198, or any combination thereof, numbered relative to GenBank Accession No. K02739, wherein when the amino acid sequence comprises a Leu at residue 14, the amino acid sequence further comprises at least one additional modification in the amino acid sequence, a mu-1 polypeptide having at least one amino acid modification, wherein the at least one amino acid modification comprises an Asp at residue 73 numbered relative to GenBank Accession No. M20161.1, a lambda-3 polypeptide having one or more amino acid modifications, wherein the one or more amino acid modifications are selected from the group consisting of a Val at residue 214, an Ala at residue 267, a Thr at residue 557, a Lys at residue 755, a Met at residue 756, a Pro at residue 926, a Pro at residue 963, a Leu at residue 979, an Arg at residue 1045, a Val at residue 1071, or any combination thereof, numbered relative to GenBank Accession No. M24734.1, wherein when the amino acid sequence comprises a Val at residue 214 or a Val at residue 1071, the amino acid sequence further comprises at least one additional modification in the amino acid sequence, or a mu-2 polypeptide having at least one amino acid modification, wherein the at least one amino acid modification comprises a Ser at residue 528 numbered relative to GenBank Accession No. AF461684.1. Alternatively or additionally, the reovirus can include a L1 genome segment comprising one or more nucleic acid modifications, an S4 genome segment comprising one or more nucleic acid modifications, an M1 genome segment comprising one or more nucleic acid modifications, or any combination thereof. Optionally, the reovirus comprises one or more of a S4 genome segment having one or more nucleic acid modifications, wherein the one or more nucleic acid modifications in the S4 genome segment are selected from the group consisting of an A at position 74 and an A at position 624, numbered relative to GenBank Accession No. K02739, a M2 genome segment having at least one nucleic acid modification, wherein the at least one nucleic acid modification comprises a C at position 248, numbered relative to GenBank Accession No. M20161.1, a L1 genome segment comprising one or more nucleic acid modifications, wherein the one or more nucleic acid modifications are selected from the group consisting of a T at position 660, a G at position 817, an A at position 1687, a G at position 2283, an ATG at positions 2284-2286, a C at position 2794, a C at position 2905, a C at position 2953, an A at position 3153, a G at position 3231, numbered relative to GenBank Accession No. M24734.1, or a M1 genome segment having at least one nucleic acid modification, wherein the at least one nucleic acid modification comprises a T at position 1595, numbered relative to GenBank Accession No. AF461684.1.

Optionally, the oncolytic virus is a recombinant oncolytic virus and/or a modified oncolytic virus. Optionally, the modified oncolytic virus does not inhibit double-stranded RNA activated protein kinase (PKR).

The provided kits can include more than one dose of the oncolytic virus. Optionally, each dose of oncolytic virus comprises approximately 10³ to 10¹² plaque forming units (PFU) of the oncolytic virus. Optionally, each dose comprises approximately 10⁸ to 10¹² PFU of the oncolytic virus. Optionally, each dose comprises approximately 10⁸ to 10¹² TCID₅₀ of the oncolytic virus. Optionally, each dose comprises approximately 1X10¹⁰ to 3X10¹⁰ TCID₅₀ of the oncolytic virus.

The provided kits can further include one or more additional therapeutic agents. Suitable additional therapeutic agents include, but are not limited to, analgesics, anesthetics, analeptics, corticosteroids, anticholinergic agents, anticholinesterases, anticonvulsants, antineoplastic agents, allosteric inhibitors, anabolic steroids, antirheumatic agents, psychotherapeutic agents, neural blocking agents, anti-inflammatory agents, antihelmintics, antibiotics, anticoagulants, antifungals, antihistamines, antimuscarinic agents, antimycobacterial agents, antiprotozoal agents, antiviral agents, dopaminergics, hematological agents, immunological agents, muscarinics, protease inhibitors, vitamins, growth factors, and hormones.

As used herein the terms treatment, treat, treating or ameliorating refers to a method of reducing the effects of a disease or condition or symptom of the disease or condition. Thus in the disclosed method, treatment can refer to a 10%, 20%, 30%, 40%, 50%, 60%, 70%, 80%, 90% or 100% reduction or amelioration in the severity of an established disease or condition or symptom of the disease or condition. For example, the method for treating cancer is considered to be a treatment if there is a 10% reduction in one or more symptoms of the disease in a subject as compared to control. Thus the reduction can be a 10, 20, 30, 40, 50, 60, 70, 80, 90, 100% or any percent reduction in between 10 and 100 as compared to native or control levels. It is understood that treatment does not necessarily refer to a cure or complete ablation of the disease, condition or symptoms of the disease or condition.

As used herein, the term subject can be a vertebrate, more specifically a mammal (e.g., a human, horse, pig, rabbit, dog, sheep, goat, non-human primate, cow, cat, guinea pig or rodent), a fish, a bird or a reptile or an amphibian. The term does not denote a particular age or sex. Thus, adult and newborn subjects, whether male or female, are intended to be covered. As used herein, patient or subject may be used interchangeably and can refer to a subject with a disease or disorder. The term patient or subject includes human and veterinary subjects.

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if an inhibitor is disclosed and discussed and a number of modifications that can be made to a number of molecules including the inhibitor are discussed, each and every combination and permutation of the inhibitor, and the modifications that are possible are specifically contemplated unless specifically indicated to the contrary. Likewise, any subset or combination of these is also specifically contemplated and disclosed. This concept applies to all aspects of this disclosure including, but not limited to, steps in methods of using the disclosed compositions. Thus, if there are a variety of additional steps that can be performed it is understood that each of these additional steps can be performed with any specific method steps or combination of method steps of the disclosed methods, and that each such combination or subset of combinations is specifically contemplated and should be considered disclosed.

A number of aspects have been described. Nevertheless, it will be understood that various modifications may be made. Furthermore, when one characteristic or step is described it can be combined with any other characteristic or step herein even if the combination is not explicitly stated. Accordingly, other aspects are within the scope of the claims.

### Example

### Example 1. Oncolytic Virus Therapy Allows for Increased Treatment Regimen of a Chemotherapeutic Agent in Pancreatic Cancer.

Described herein are the results of a study of carboplatin, paclitaxel and reovirus serotype-3 dearing strain (Reolysin®) administration as compared to carboplatin and paclitaxel in patients with recurrent or metastatic pancreatic cancer. Patients in Group I were given paclitaxel as an intravenous infusion over 3 hours and carboplatin as an intravenous infusion over 30 minutes on day 1 of a 21 day cycle and Reolysin® as an intravenous infusion over 60 minutes on days 1, 2, 3, 4, and 5 of a 21 day cycle. This cycle was repeated every 21 days in the absence of disease progression or unacceptable toxicity. Patients in Group II were given paclitaxel as an intravenous infusion over 3 hours and carboplatin as an intravenous infusion over 30 minutes on day 1 of a 21 day cycle. This cycle was repeated every 21 days in the absence of disease progression or unacceptable toxicity. Patients in Group I received more cycles of treatment than patients in Group II. Thus, in the presence of reovirus patients were capable of being treated with increased number of cycles of treatment and thus an increased treatment regimen as compared to patients that were not given reovirus, i.e., in the absence of reovirus.

### Example 2. Oncolytic Virus Therapy Allows for Increased Treatment Regimen of a Chemotherapeutic Agent in Head and Neck Cancer.

Described herein are the results of a study of carboplatin, paclitaxel and reovirus serotype-3 dearing strain (Reolysin®) administration as compared to carboplatin and paclitaxel in patients with platinum refractory head and neck cancer. Patients in Group I were given 175 mg/m² paclitaxel as an intravenous infusion over 3 hours and 5AUC mg/mL carboplatin as an intravenous infusion over 30 minutes on day 1 of a 21 day cycle and 3X10¹⁰ TCID₅₀ of Reolysin® as an intravenous infusion over 60 minutes on days 1, 2, 3, 4, and 5 of a 21 day cycle. This cycle was repeated every 21 days in the absence of disease progression or unacceptable toxicity. Patients in Group II were given 175 mg/m² paclitaxel as an intravenous infusion over 3 hours and 5AUC mg/mL carboplatin as an intravenous infusion over 30 minutes on day 1 of a 21 day cycle. This cycle was repeated every 21 days in the absence of disease progression or unacceptable toxicity. Five (5) patients (6.02%) in Group II had dose reductions due to neuropathy while no patients (0%) in Group I had dose reductions due to neuropathy (p=0.0245). Thus, the patients in Group I received an increased treatment regimen as compared to patients in Group II. Thus, in the presence of Reolysin® patients were capable of being treated with increased treatment regimens as compared to patients that were not given Reolysin®.

## Claims

1. An oncolytic virus for use in a method of treating cancer in a subject, the method comprising administering to the subject the oncolytic virus and an increased number of treatment cycles with a chemotherapeutic agent as compared to standard therapy, wherein the increased number of treatment cycles comprises 9, 10, 11, 12, 13, 14, 15, 16, 17, 18, 19, 20, 21, 22, 23, 24, 25 or more treatment cycles with the chemotherapeutic agent, and wherein the oncolytic virus is a reovirus having IDAC Accession No. 190907-01.

2. The oncolytic virus for use of claim 1, wherein the chemotherapeutic agent is a platinum compound.

3. The oncolytic virus for use of claim 2, wherein the platinum compound is selected from the group consisting of cisplatin, carboplatin, oxaliplatin, satraplatin, picoplatin, nedaplatin, and triplatin.

4. The oncolytic virus for use of claim 1, wherein the chemotherapeutic agent is a taxane.

5. The oncolytic virus for use of claim 4, wherein the taxane is selected from the group consisting of cabazitaxel, abraxane, paclitaxel and docetaxel.

6. The oncolytic virus for use of any one of claims 1-5, wherein the chemotherapeutic agent is a platinum compound and a taxane.

7. The oncolytic virus for use of any one of claims 1-6, wherein approximately 10³ to 10¹² plaque forming units (PFU) of the oncolytic virus is administered to the subject; or
wherein approximately 10⁸ to 10¹² PFU of the oncolytic virus is administered to the subject; or
wherein approximately 10⁸ to 10¹² TCID₅₀ of the oncolytic virus is administered to the subject.

8. The oncolytic virus for use of any one of claims 1-7, wherein the cancer is ras-activated.

## Patentansprüche

1. Onkolytisches Virus, das bei einem Patienten in einem Krebsbehandlungsverfahren zur Anwendung kommt, wobei das Verfahren umfasst, dem Patienten das onkolytische Virus zu verabreichen sowie den Patienten im Vergleich zur Standardtherapie einer erhöhten Anzahl von Behandlungszyklen mit einem Chemotherapeutikum zu unterziehen, wobei die erhöhte Anzahl von Behandlungszyklen 9, 10, 11, 12, 13, 14, 15, 16, 17, 18, 19, 20, 21, 22, 23, 24, 25 oder mehr Behandlungszyklen mit dem Chemotherapeutikum umfasst, und wobei das onkolytische Virus ein Reovirus mit der IDAC-Zugangsnummer 190907-01 ist.

2. Onkolytisches Virus gemäß Anspruch 1, wobei das Chemotherapeutikum eine Platinverbindung ist.

3. Onkolytisches Virus gemäß Anspruch 2, wobei die Platinverbindung ausgewählt ist aus der Gruppe bestehend aus Cisplatin, Carboplatin, Oxaliplatin, Satraplatin, Picoplatin, Nedaplatin und Triplatin.

4. Onkolytisches Virus gemäß Anspruch 1, wobei das Chemotherapeutikum Taxan ist.

5. Onkolytisches Virus gemäß Anspruch 4, wobei das Taxan ausgewählt ist aus der Gruppe bestehend aus Cabazitaxel, Abraxane, Paclitaxel und Docetaxel.

6. Onkolytisches Virus gemäß einem der Ansprüche 1 bis 5, wobei das Chemotherapeutikum eine Platinverbindung und ein Taxan ist.

7. Onkolytisches Virus gemäß einem der Ansprüche 1 bis 6, wobei dem Patienten ungefähr 10³ bis 10¹² plaquebildende Einheiten (PFU) des onkolytischen Virus verabreicht werden; oder
wobei dem Patienten ungefähr 10⁸ bis 10¹² plaquebildende Einheiten des onkolytischen Virus verabreicht werden; oder
wobei dem Patienten ungefähr 10⁸ bis 10¹² TCID₅₀ des onkolytischen Virus verabreicht werden.

8. Onkolytisches Virus gemäß einem der Ansprüche 1 bis 7, wobei der Krebs Rasaktiviert ist.

## Revendications

1. Un virus oncolytique pour utilisation dans un procédé de traitement du cancer chez un sujet, le procédé comprenant l'administration au sujet du virus oncolytique et un nombre accru de cycles de traitement avec un agent chimiothérapeutique par rapport à la thérapie standard, dans lequel le nombre accru de cycles de traitement comprend 9, 10, 11, 12, 13, 14, 15, 16, 17, 18, 19, 20, 21, 22, 23, 24, 25 cycles de traitement ou plus avec l'agent chimiothérapeutique, et dans lequel le virus oncolytique est un réovi-rus ayant l'adhésion ADIC n° 190907-01.

2. Le virus oncolytique pour utilisation selon la revendication 1, dans lequel l'agent chimiothérapeutique est un composé de platine.

3. Le virus oncolytique pour utilisation selon la revendication 2, dans lequel le composé de platine est choisi dans le groupe constitué par le cisplatine, le carboplatine, l'oxali-platine, le satraplatine, le picoplatine, le nédaplatine, et le triplatine.

4. Le virus oncolytique pour utilisation selon la revendication 1, dans lequel l'agent chimiothérapeutique est un taxane.

5. Le virus oncolytique pour utilisation selon la revendication 4, dans lequel le taxane est choisi dans le groupe constitué par le cabazitaxel, l'abraxane, le paclitaxel et le docé-taxel.

6. Le virus oncolytique pour utilisation selon l'une quelconque des revendications 1 à 5, dans lequel l'agent chimiothérapeutique est un composé de platine et un taxane.

7. Le virus oncolytique pour utilisation selon l'une quelconque des revendications 1 à 6, dans lequel environ 10³ à 10¹² unités formatrices de plaque (UFP) du virus oncolytique sont administrées au sujet ; ou
dans lequel environ 10⁸ à 10¹² UFP du virus oncolytique sont administrés au sujet ; ou
dans lequel environ 10⁸ à 10¹² DICT₅₀ du virus oncolytique sont administrées au sujet.

8. Le virus oncolytique pour utilisation selon l'une quelconque des revendications 1 à 7, dans lequel le cancer est activé par ras.
